# EUROPEAN PATENT APPLICATION

(11) **EP 1 374 833 A1**
(43) Date of publication of application: **02.01.2004**
(21) Application number: 02704768.7
(22) Date of filing: 28.02.2002
(51) Int. Cl.: A61K 7/00

(54) **USE OF CERAMIDE-RICH LANOLIN FRACTIONS IN SKIN TREATMENT AND COMPOSITIONS CONTAINING SAID FRACTIONS**

(30) Priority: 02.03.2001 ES 200100508
(71) Applicant: CONSEJO SUPERIOR DE INVESTIGACIONES CIENTIFICAS, 28006 Madrid (ES)
(72) Inventor: AODERCH NEGRA, Maria, Luisa, C/Jordi Girona 18 (ES); FONOLLOSA ARGEM , Jordi, C/Jordi Girona, 18 (ES); PERA L PEZ, Montserrat, de, C/Jordi Girona 18 (ES); MART GELABERT, Meritxell, C/Jordi Girona, 18 (ES); MAZA RIBERA, Alfonso, de, la, C/Jordi Girona 18 (ES); BARRA JUEZ, José, Luis, C/Jordi Girona, 18-2 (ES)
(74) Representative: Carpintero Lopez, Francisco
(86) International application number: PCT/ES2002/000090
(87) International publication number: WO 2002/069912

(57) **Abstract**

The discovery that lanolin contains ceramides, albeit in small proportions, has made it possible to obtain lanolin fractions with relative high contents of natural ceramides from wool. Said lanolin fractions have a moisturizing and protective activity in the human skin that resembles at least that obtained with synthetic ceramides currently used in cosmetic and dermatological products to enhance the skin protective barrier function of the stratum corneum. Hence, said fractions can be used in the preparation of cosmetic and/or pharmaceutical compositions for the treatment of the human skin.

## Description

### Field of the art

The present invention relates to the ceramide-rich lanolin fractions and to the compositions and use thereof in the preparation of cosmetic and pharmaceutical products for the treatment and conditioning of the skin.

### Previous state of the art

The term ceramide is assigned to natural or synthetic lipidic structures such as those described by Downing D.T. et al. in The Journal of Investigative Dermatology 84:410-412 (1985). These ceramides are fatty amides of sphyngosin or phytosphyngosin and are usually grouped into 6 types, as mentioned in the paper by Downing et al.

Ceramides in the stratum corneum of the skin form an important part of the lipidic barrier of the human skin, which is responsible for protecting the skin against external aggression and for maintaining the necessary hydration balance for good skin preservation.

The use of ceramides in different products for skin care and treatment is well known. Here, it is mentioned, by way of example, the description of the US patent US-A-5830481. However, commercially available products for the care and treatment of skin use mainly synthetic ceramides.

A number of extracts of products naturally rich in ceramides and their application for skin care have been described, e.g. an extract of soya lecithin in patent application FR-A-2753200, an extract from silk worm larvae in patent application PCT WO92/13543 and extracts of mulberry leaves in patent applications FR-A-2739853 and WO-A-98/46558.

The use of extracts from ceramide-rich internal wool lipids is described, in our application for the Spanish patent n° P9901541. It should be pointed out that these internal wool lipids do not proceed from and have no direct relationship with lanolin.

Lanolin is a group of lipids that are found on the surface of wool fibres. These lipids are secreted by the sebaceous glands of the sheep and are deposited on the surface of the fibres by means of simple contact. There is no relationship between these and the lipids of the internal structures of the wool fibres, which are of endogenous origin.

It is well known that lanolin and some of its fractions are used in cosmetic and pharmaceutical products for skin care although, to date, the authors of the present invention do not have any evidence that certain fractions of lanolin have been reported to contain relatively high proportions of natural ceramides that are able to enhance the natural protective activity of the stratum corneum layer of the skin more effectively than lanolin itself. This is used in cosmetic only as an additional substrate to preparations that can also contain ceramides of synthetic origin.

In patent US2758125, a lanolin fraction called "lanolin oil" is described. This fraction is obtained by dissolving lanolin in a mixture of hot solvents, for example, in a mixture of n-heptane and methylethylketone, eliminating the fatty mass that separates on cooling and isolating the light fraction by evaporation of solvents. This patent describes how the "lanolin oil" obtained can be used in cosmetic preparations, although it does not give a detailed description of its components. In patents US3052608, US3666857 and US4279262 a reference is also made to the cosmetic use of "lanolin oil" described in the aforementioned patent US2758152.

In patent US4091035, the fractionation of lanolin by dissolving it in n-hexane and extracting the solution obtained with ethanol at 93% is described. In this case, the polar fraction is specifically ruled out for cosmetic use. In patent US4138416, the polar fractions of lanolin are eliminated by eluting a solution of this in hexane using a column equipped with a filling able to retain the polar components. The useful fraction for cosmetic purposes is that obtained by evaporation of hexane.

In patent US4165327 fractionation of lanolin is done by extraction with a mixture of methanol and water from a solution of lanolin in hexane. In this case, only the apolar fraction is used for cosmetic purposes and the polar fraction is discarded.

Finally, patents US4960592 and US5641809 describe the cosmetic use of a mixture of lanolin and lanolin oil obtained by fractionated crystallisation of lanolin in solvents, which is used in the previously mentioned patent US2758125. These patents include a detailed description of the components of lanolin and lanolin oil but make no reference to ceramides.

Analyses of the fractions of commercially available "lanolin oil" or "lanolin alcohols" show that these contain very small amounts of ceramides, in general lower than 3% by weight.

Hence, in the state of the art described above there is no specific mention that selected lanolin fractions contain ceramides and no explicit or implicit mention of the cosmetic use of lanolin fractions with a relatively high content of ceramides. In fact, from the contents of several of the patents mentioned the expert in the art can discard "a priori" cosmetic use of the polar fractions of lanolin, such as those produced by extraction with organic solvents or aqueous-organic solvents of a lanolin solution in a clearly apolar solvent such as hexane.

Given the current predominance of the use of synthetic ceramides in the preparation of products for skin care and treatment, there is a need to find new natural extracts rich in ceramides from cheap and easily available materials. One such extract is provided by lanolin, a usual by-product of the wool fibre industry, which is especially cheap and available.

### Aim of the invention

The aim of the present invention is the use of ceramide-rich lanolin fractions to prepare products for skin care and treatment.

Part of the aim of the present invention also concerns the cosmetic and pharmaceutical compositions that contain lanolin fractions rich in ceramides.

### Description of the invention

The authors of the present invention have discovered that lanolin contains natural ceramides in relatively low proportions and that, fractions rich in these natural ceramides can be obtained from lanolin. These lanolin fractions confer properties of protection and hydration to the skin. These properties are at least similar to those conferred by ceramides that are currently used to date, rendering them suitable for application in the preparation of cosmetic and dermatological products.

Given that lanolin is a very cheap and easily available natural product, this invention may replace synthetic ceramides and natural extracts used to date in the preparation of these formulations.

For the purpose of the present invention, a ceramide-enriched lanolin fraction is one that contains at least 4% by weight, preferably at least 5% by weight and even more preferably at least 10% by weight of natural ceramides from wool.

The ceramide-enriched lanolin fractions can be obtained by extracting a lanolin solution in an apolar solvent with an alcoholic or an aqueous-alcoholic solvent. The alcoholic or aqueous-alcoholic fraction of the lanolin, after eliminating the solvent by conventional methods, for example by evaporation, is the part that is useful for the purpose of this invention owing to the ceramide levels present.

The apolar solvent can be an aliphatic hydrocarbonated solvent, preferably n-hexane or n-heptane, and the proportion of lanolin in the apolar solvent can range between 10 and 100 g/L, preferably between 20 and 80 g/L.

The alcoholic solvent can be an aliphatic alcohol with a chain of up to eight carbons either in a straight or a branched chain, preferably methanol, ethanol, isopropanol, n-propanol or n-butanol, most preferably methanol or ethanol. The proportion of water in the case of an aqueous-alcoholic solvent can range from 5% (v/v) to 40% (v/v), preferably between 10% (v/v) and 30% (v/v).

The extraction temperature can range from 0° C to 40° C, and preferably between 15° C and 25° C.

Especially preferable are fractionation methods that involve dissolving the lanolin in n-hexane, proceeding to the extraction of the hexanic solvent, at near room temperature, with methanol or a mixture of methanol and water 4:1 (v/v), and finally, eliminating the methanolic or aqueous-methanolic solvent by evaporating it.

When methanol is used as an extraction solvent, lanolin fractions that contain around 5% by weight of ceramides and represent 15-20% by weight of the initial lanolin content can be obtained, whereas with a mixture of methanol/water of 4:1 (v/v), fractions that contain around 15% by weight of ceramides and represent 2.0% - 2.5% by weight of the initial lanolin content can be obtained.

The elimination of aqueous or aqueous/alcoholic solvents from solutions obtained during the extraction process generates ceramide-enriched lanolin fractions suitable for the object of the present invention, which can be used either in the form of oily solutions in physiologically acceptable lipophylic solvents or as liposomal structures, for example similar to those described in the paper by L. Coderch et al. *J. Am. Oil Chem.* Soc. 73:1713-1718 (1996), although the liposomal forms are preferable because of their stability, ease of handling and formulation, and their excellent skin absorption properties.

Trials in which the lanolin fractions so obtained were applied to the skin -which will be explained in greater detail in the section of Examples-demonstrate that these fractions present a good ability to reduce the transepidermal water loss (TEWL) and an improvement in the hydration capacity of the skin, especially when this is subject to conditions of chemical aggression, with values clearly comparable to those obtained with commercial ceramides that imitate the nature of lipids of the stratum corneum.

Therefore, lanolin fractions that correspond to the object of this invention can be advantageously incorporated, in different proportions, as essential ingredients of pharmaceutical and/or cosmetic compositions for skin care and treatment.

The said pharmaceutical and/or cosmetic compositions can be any of the types known by experts in the art for topical applications, such as pomades, ointments, creams, emulsions, lotions, dyes, etc. which can be prepared by combining lanolin fractions with physiologically acceptable excipients and other suitable finishing compounds, using well known techniques such as mixing, dissolving, emulsion, etc. As a reference, for example the types of compositions and additional ingredients described in the US patent US-A-5830481 could be appropriate.

The compositions obtained in this way show an excellent capacity for reinforcing the protective lipidic barrier of human skin and for appreciably improving its degree of hydration.

The following examples provide experts in the art with a sufficiently clear and complete explanation of the present invention, but they should not be considered as limitations on the essential aspects of the object of the invention, as mentioned in previous sections.

### Examples

### Example 1.- Extraction of a ceramide-enriched lanolin fraction with methanol.

A total of 60 g of lanolin are dissolved at room temperature in 1 L of n-hexane and the lanolin hexanic solution is placed in the appropriate separating funnel together with 250 mL of methanol, which is then shaken vigorously for five minutes. The separating funnel is left to stand so that the phases decant and the lower methanolic phase is collected. The upper hexanic phase is extracted twice more with 250 mL of methanol each time and the hexanic phase is discarded.

The methanol is eliminated with a rotavapor and a lanolin fraction weighing between 9 and 12 g is obtained, depending on the degree of exhaustion of the solvent.

### Example 2.- Extraction of a ceramide-enriched lanolin fraction with methanol/water

At room temperature, 20 g of lanolin are dissolved in 1 L of n-hexane and the lanolinic hexanic solution is placed in an appropriate separating funnel together with 250 mL of methanol/water 4:1 (v/v), and shaken vigorously for five minutes. The separating funnel is left to stand until the phases have separated and the lower aqueous/methanolic phase is collected. The upper hexanic phase is extracted twice more with 250 mL of methanol/water 4:1 (v/v) each time and the aqueous/methanolic phases are combined and the hexanic phase is discarded.

The methanol and water are eliminated with a rotavapor, and a fraction of lanolin weighing between 0.4 and 0.5 g is obtained, depending on the degree of exhaustion of the solvent.

### Example 3.- Ceramide content of the lanolin fractions

Lipids were quantified using a TLC/FID IATROSCAN MK-5 automatic analyser (latron Lab. Ink. Tokyo, Japan). The working conditions selected for the apparatus are those recommended in the literature to obtain optimum results: air flow: 2000 mL/min; hydrogen flow: 160-180 mL/min; sweep rate: 2-3 mm/s. The data are processed by the BOREAL v.2.5 software.

The lanolin fractions are deposited directly using an automatic applicator (Sample spotter SES 3202/IS-01, Germany) on silica columns (Silica gel SIII Chromarods) and are eluted vertically to 10 cm by three solvent systems in order of increasing polarity. The hexane/diethylic ether/formic acid system (47:23:0,3) can be employed to separate the apolar lipids and quantify them using a partial sweep of 85%. The chloroform/hexane/acetone/methanol system (55:5:7:3) and subsequently that of chloroform/hexane/acetone/methanol (50:10:9:1) can be used to separate and quantify the remaining polar lipids. After each elution, the solvent is eliminated by heating to 60° C, followed by cooling in a chamber at room temperature and dry conditions. The same procedure was applied to the standards (palmitic acid and cholesterol of Fluka Chemicals Co. (Buchs, Switzerland); cholesterol ester, 7OH-cholesterol, triglycerides, diglycerides, galactoceramide, type II and type IV ceramide of Sigma Chemicals Co. (St. Louis, MO, US); type III and type VI (b) ceramide of Cosmoferm (Delft, Netherlands) to determine their calibration curves and then quantify each component.

The method described is used to analyse the following products derived from lanolin:

| | |
|---|---|
| LAN-1 | Anhydrous lanolin commercialised by the company Lanolines Stella under the brand name STELLUX Al. |
| LAN-2 | Unrefined lanolin used as a starting material in Examples 1 and 2. |
| LOI-1 | "Lanolin oil" commercialised by the company Lanolines Stella under the brand name STELLANOL. |
| LOI-2 | "Lanolin oil" commercialised by the company Lanolines de la Tossee under the brand name LANOR CRYSTAL. |
| LOL-1 | "Lanolin alcohols" commercialised by Lanolines de la Tossee under the brand name LANOR A. |
| LC-M | Lanolin fraction obtained following the methodology described in Example 1. |
| LC-MA | Lanolin fraction obtained as described in Example 2. |

The results obtained are shown in Table 1 in which the numerical results are expressed as % by weight of the total weight of sample analysed.

**Table 1.-**

| Analysis of lanolin fractions | | | | | | | |
|---|---|---|---|---|---|---|---|
| | LAN-1 | LAN-2 | LOI-1 | LOI-2 | LOL-1 | LC-M | LC-MA |
| Waxes | 54.4 | 48.4 | 43.6 | 48.9 | 1.8 | 34.5 | 3.2 |
| Fatty acids | 12.7 | 10.3 | 14.9 | 20.7 | 10.0 | 11.7 | 2.3 |
| Fatty alcohols | 6.1 | 4.4 | 4.4 | 5.8 | 43.9 | 14.6 | 1.4 |
| Sterols | 3.4 | 2.6 | 3.4 | 3.9 | 30.6 | 6.3 | 8.2 |
| Ceramide II | 0.4 | 1.1 | 0.5 | 0.5 | 0 | 1.7 | 5.7 |
| 7OH-cholesterol | 1.1 | 1.3 | 1.2 | 1.3 | 4.4 | 2.4 | 6.0 |
| Ceramide III/IV | 0.8 | 1.0 | 0.8 | 0.9 | 1.2 | 1.8 | 5.3 |
| Ceramide VI (b) | 1.0 | 1.7 | 1.1 | 1.2 | 1.3 | 2.3 | 4.8 |
| Cerebrosides | 2.2 | 2.6 | 2.4 | 3.1 | 2.5 | 4.1 | 5.9 |
| % lipid analysed | 82.1 | 73.3 | 72.3 | 86.3 | 95.6 | 79.3 | 42.8 |
| % Ceramides | 2.3 | 3.7 | 2.4 | 2.6 | 2.48 | 5.8 | 15.8 |

The % of lipid analysed corresponds to the total of lipids analysable with the method used. The remaining ones, up to 100%, correspond to cholesterol derivatives, some unknown ones, to polar compounds, protein fractions etc.

From the results recorded in the table the following deductions can be made:
- Industrial lanolin contains ceramides in significant, although relatively low, proportions (less than 4% by weight).
- Commercially available "lanolin oil" or "lanolin alcohol" fractions present a low ceramide content similar to that of lanolin.
- The lanolin fractions appropriate for the purposes of this invention (Examples 1 and 2) present a markedly higher ceramide content than lanolin and the previously mentioned commercial fractions, of the order of 5% or more. Especially noteworthy is the high ceramide content of the fractions obtained by aqueous-alcoholic extraction, which can exceed 15% by weight.

### Example 4.- Procedure for obtaining liposomal forms suitable for dermatological application.

General technique.- The lipidic sample dissolved in chloroform/methanol 2:1 (v/v) is evaporated to dryness in a nitrogen atmosphere and the film formed on the walls of the recipient is resuspended with an appropriate volume of NaCl solution at 0.9% by weight to obtain a suspension of lipid concentration of 25 mg/mL. The formation of multilammelar liposomes is carried out by sonicating the sample at 65° C, a temperature higher than that of the transition phases of the implicated lipids, for 15 minutes in a LABSONIC 1510 sonicator (B. Braun, Germany).

After leaving to stand for 24 h at room temperature, unilammelar liposomes of a specific size are obtained by extruding the multilammelar liposome suspension three times at 65°C through filters with a pore size 800, 400 and 200 nm (MILLIPORE, Ireland), providing a suspension of liposomes with a size of approximately 200 nm by means of the Light Scattering technique, using a photo-correlation spectrometer AUTOSIZER IIc (Malvern, UK).

With the aid of the general technique described here the following three liposomal samples are prepared:
A liposomes: This consists of a lipidic composition that imitates that of the stratum corneum of the human skin using a commercial ceramide. They are prepared by combining a mixture of 40% ceramides III (Cosmoferm, Delft, Holland), 25% cholesterol, 25% palmitic acid and 10% cholesterol sulphate (Fluka Chemicals Co., Buchs, Switzerland).
B liposomes: This consists of 40% of the lanolin LC-MA fraction obtained in Example 2, 25% cholesterol, 25% palmitic acid and 10 % cholesterol sulphate (Fluka Chemicals Co., Buchs, Switzerland).
C liposomes: This only consists of the LC-M lanolin fraction obtained in Example 1.

### Example 5.- Study of the efficacy in healthy human skin.

The aim of this trial is to assess the effect of topical application of liposomes formed from the ceramide-rich lanolin fractions on the barrier capacity and hydration of healthy skin, and to compare this with the effect produced by the liposomes obtained from a lipidic composition that simulates that of the stratum corneum (SC) of human skin.

The evaluation of these properties of the skin is performed using the measures made with the Tewameter® TM 210 and the Corneometer® CM 825, both from Courage & Khazaka (Germany). The Tewameter measures the transepidermal water loss (TEWL), which reflects the state of the barrier function of the skin. High values of TEWL correspond to a reduced barrier function i.e. to a skin less protected against external aggression and with a lower capacity to limit the entrance of foreign substances into the body and the release of water into the environment. The Corneometer measures the capacitance of the skin, in arbitrary units, which is directly associated with the amount of water present in the skin and is, therefore, effective in determining cutaneous hydration.

In sample efficacy studies, 7 healthy volunteers, 6 women and 1 man, aged between 25 and 57 years, participated. Over the treatment period the subjects were allowed to wash normally although they were requested to refrain from using any other skin care product on their forearms.

A total of 8 applications were made over 10 days in intact areas of the forearms of 4.4 µl/cm² of A and B liposomes, described in Example 4. A harmless and ineffective placebo was also applied. The degree of cutaneous hydration was assessed daily and always before the topical application. Similarly, TEWL was evaluated in basal conditions (day 1) and 24h after the last application (day 11).

The values obtained for skin capacitance and for TEWL are given in Tables 2 and 3, doubly relativised as percentage variation relative to baseline values and values of the untreated area or control.

**Table 2.-**

| Variation in capacitance expressed as % variation with respect to baseline and control values | | | |
|---|---|---|---|
| Day | Placebo | A liposomes | B liposomes |
| 2 | 96 | 98 | 100 |
| 3 | 104 | 106 | 102 |
| 4 | 104 | 108 | 103 |
| 5 | 105 | 107 | 108 |
| 8 | 98 | 102 | 99 |
| 9 | 97 | 105 | 102 |
| 10 | 99 | 106 | 103 |

**Table 3.-**

| Variation in TEWL at the end of treatment expressed as % of variation with respect to baseline and control values. | |
|---|---|
| Sample | TEWL |
| Placebo | 98.3 |
| A liposomes | 99.0 |
| B liposomes | 95.7 |

In the light of the results observed in Tables 2 and 3, both types of liposomes were found to produce an increased hydration in treated areas, in the order of 7-8 % after the fifth day of treatment. Also B liposomes produced a drop of 5% in TEWL, reflecting a noticeable, albeit slight, improvement in the barrier function of healthy skin.

### Example 6.- Study of the protection of human skin against detergent-induced contact dermatitis.

After the treatment described in Example 5, the areas treated with the different liposomal samples and the control area, were subjected for 24 h to an aqueous solution of sodium lauryl sulphate (SLS) using an occlusive patch. This was done by applying 50µl of a solution of SLS (Merck, Darmstadt, Germany) at 0.5% to a filter paper disc placed inside the aluminium chamber of the patch (d=12mm, Large Finn Chamber, Epitest Oy, Finland). The resulting degree of irritation was evaluated 24 h after removing the patch by measuring TEWL.

The results are expressed in Table 4 as a variation in TEWL with respect to the baseline value, taking this value as that obtained in Example 5 for day 11.

**Table 4.-**

| Variation in TEWL after exposure to SLS. | |
|---|---|
| Sample | TEWL (% baseline value) |
| Control | 222.2 |
| Placebo | 221.2 |
| A liposomes | 203.1 |
| B liposomes | 202.7 |

It can be observed that exposure to SLS affected areas that had been treated previously with liposomal solutions to a lesser degree. This could be interpreted as evidence that A and B liposomes had been incorporated into the intercellular lipids of the stratum corneum, strengthening the barrier and thus reducing the susceptibility to irritant contact dermatitis induced by SLS.

### Example 7.- Study of reparation of skin with detergent-induced contact dermatitis.

Irritant contact dermatitis was induced by applying occlusive patches with SLS to intact areas of the forearm of the seven volunteers in Example 5. In this case, 50µl of an aqueous solution of 2% SLS was applied and the patch (Large Finn Chambers, 12mm) was placed on the skin for 4 hours. TEWL was used as a parameter to determine alteration of the barrier function, which was assessed before exposure to SLS and 20 h after removing the patch.

After the chemical aggression produced by SLS, 5µl of the 3 liposomal samples described in Example 4 and the placebo were applied daily for 10 days. Measurements of TEWL were made on days 1, 3, 7 and 11 after exposure to SLS and always before applying the samples in order to study the recovery of the barrier function adversely affected by SLS.

Table 5 shows the TEWL values obtained before and after treatment with SLS, and these, as expected, show an increase after the chemical aggression.

**Table 5.-**

| Values of TEWL before and after aggression with SLS (mean ± standard deviation, n=7) | | |
|---|---|---|
| Zone | Baseline TEWL | TEWL after SLS |
| Control | 8.7 ± 1.9 | 16.5 ± 7.1 |
| Placebo | 8.8 ± 2.5 | 16.1 ± 7.5 |
| A liposomes | 8.8 ± 2.1 | 16.8 ± 7.9 |
| B liposomes | 9.6 ± 2.4 | 14.6 ± 7.2 |
| C liposomes | 9.4 ± 1.9 | 16.5 ± 7.3 |

In the study of the recovery of the barrier function, values of TEWL were doubly relativised so that 100% indicates the aggression produced by SLS and 0% the baseline state, i.e., complete recovery. The results are shown in Table 6.

**Table 6.-**

| Recovery of TEWL over the treatment period. Variation in % TEWL with respect to baseline and control values. | | | |
|---|---|---|---|
| | Day 3 | Day 7 | Day 11 |
| Control | 66.7 | 44.9 | 29.5 |
| Placebo | 89.0 | 53.4 | 32.9 |
| A liposomes | 56.3 | 37.5 | 21.3 |
| B liposomes | 64.0 | 42.0 | 24.0 |
| C liposomes | 73.2 | 40.8 | 22.5 |

Over 10 days of studies after SLS exposure, there was a progressive reduction in TEWL values. It is noteworthy that in areas treated with the three types of liposomes there is an acceleration in the recovery of the barrier function with respect to the physiological reparation observed in the untreated area (control). In contrast, topical application of the placebo reduces the speed of reparation of the cutaneous barrier.

In summary, the following conclusions can be drawn from the results obtained in Examples 5 to 7:
- Daily topical applications of the ceramide-rich lanolin fractions in healthy human skin lead to an increase in the hydration of the skin and enhances the protective action by reducing the skin's susceptibility to the irritant stimulus by 20%.
- The said lanolin fractions show a capacity for accelerating the reparative process of the barrier function of the skin adversely affected by a detergent, at least in a way similar to that of conventional ceramides used to date.
- In general, ceramide-rich lanolin fractions (B liposomes and C) produce effects similar to those obtained with synthetic ceramides (A liposomes) when applied topically in healthy skins and in skins affected by a detergent.

## Claims

1. The use of lanolin fractions that contain at least 4% by weight of natural ceramides from wool in the manufacture of cosmetic and/or pharmaceutical compositions for the care and treatment of human skin.

2. The use, according to Claim 1, **characterised in that** the lanolin fractions contain at least 10% by weight of natural ceramides from wool.

3. The use according to Claims 1 and 2, **characterised in that** the lanolin fractions contain at least 15% by weight of natural ceramides from wool.

4. A cosmetic and/or pharmaceutical composition for the treatment of human skin **characterised in that** it includes a lanolin fraction that contains at least 4% by weight of natural wool ceramides.

5. A composition according to Claim 4, **characterised in that** it includes a lanolin fraction that contains at least 10% by weight of natural ceramides from wool.

6. A composition according to Claims 4 and 5, **characterised in that** it includes a lanolin fraction that contains at least 15% by weight of natural ceramides from wool.
